# EUROPEAN PATENT APPLICATION

(11) **EP 3 338 806 A1**
(43) Date of publication of application: **27.06.2018**
(21) Application number: 16205708.7
(22) Date of filing: 21.12.2016
(51) Int. Cl.: A61K 47/69, A61K 49/00, A61P 35/00, A61K 49/04, B82Y 5/00

(54) **METHOD FOR FUNCTIONALISING NANOPARTICLES**

(71) Applicant: Université de Namur, 5000 Namur (BE)
(72) Inventor: BONIFAZI, Davide, Cardiff, CF23 5AT (GB); CORVAGLIA, Valentina, 00013 Mentana (Roma) (IT); MAREGA, Riccardo, 5000 NAMUR (BE); LUCAS, Stéphane, 5380 FORVILLE (BE)
(74) Representative: Pronovem

(57) **Abstract**

The present invention relates to methods for functionalizing nanoparticles with organic molecules comprising a carboxyl group, such as antibodies or proteins. The functionalized nanoparticles obtained according to methods of the present invention can be used in a wide variety of medical and biological applications.

## Description

The present invention relates to methods for functionalizing nanoparticles with organic molecules such as antibodies or proteins. The functionalized nanoparticles obtained according to methods of the present invention can be used in a wide variety of medical and biological applications.

In the art, various methods for nanoparticle functionalization are already available. In these methods, often, antibodies or other types of biological molecules are bound to (metal-based) nanoparticles through carbodiimide chemistry, i.e. using EDC (1-ethyl-3-(3-dimethylaminopropyl)carbodiimide) and/or NHS (*N*-hydroxysuccinimide).

Davis M. E. et al., for example, describe in Bioconjugate Chem. 2015, 26, 812-816 the assembly of antibody containing gold nanoparticles. Conjugates of polyethylene glycol (PEG) and *inter alia* Cetuximab were prepared by antibody reaction with NHS-PEG-OPSS (with OPPS referring to ortho-pyridyl disulphide or ortho pyridyldithio). More specifically, NHS-PEG-OPSS reacts with the amine groups of antibodies to yield antibody-PEG conjugates through amide bond formation. Purified antibody conjugates were then combined with mPEG-SH and assembled onto the surface of the gold nanoparticles. The ability of the prepared nanoparticles to produce an antibody-dependent cellular cytotoxicity (ADCC) effect *in vivo* was subsequently investigated.

WO 2007/122259, for example, describes the functionalization of nanoparticles with spacers such as polyethylene oxide (PEO) using EDC/NHS chemistry as well, the spacer itself being linked to a protein in a stereo-specific manner, thereby ensuring controlled orientation of the particle-bound protein.

The known methods, however, have the drawback that the chemical reactions involved are complex, comprising multiple steps, and take a long time to complete the coupling reaction (requiring numerous chemical reagents and high amounts thereof). Furthermore, the chemical compounds used in the prior art methods show several disadvantages in terms of water solubility and stability in buffers (i.e. carbodiimides), and possibility of detonation (benzothiazole-based). Hence, these methods are not environmentally friendly, laborious and costly.

An objective of aspects of the present invention is to provide an improved method for functionalizing nanoparticles. It is an object to provide such methods which allow a faster and more efficient functionalization of nanoparticles compared to prior art methods. It is also an object to provide such methods which are easier to implement and which are more cost effective. It is a further object to provide such methods using more versatile chemical compounds. It is hence an object of the present invention to provide methods for functionalizing nanoparticles, which are less laborious, more environmentally friendly, and/or more economical compared to prior art methods.

According to an aspect of the invention, there is therefore provided a method for functionalizing nanoparticles, as set out in the appended claims.

According to another aspect of the invention, there are provided functionalized nanoparticles, or there is provided a colloidal suspension comprising functionalized nanoparticles, obtainable by a method according to the invention, as set out in the appended claims.

According to yet another aspect of the invention, there are provided functionalized nanoparticles, or there is provided a colloidal suspension comprising functionalized nanoparticles, obtainable by a method according to the invention, for use in therapy or *in vivo* diagnostics, as set out in the appended claims.

Advantageous aspects of the present invention are set out in the dependent claims.

Aspects of the invention will now be described in more detail with reference to the appended drawings wherein:

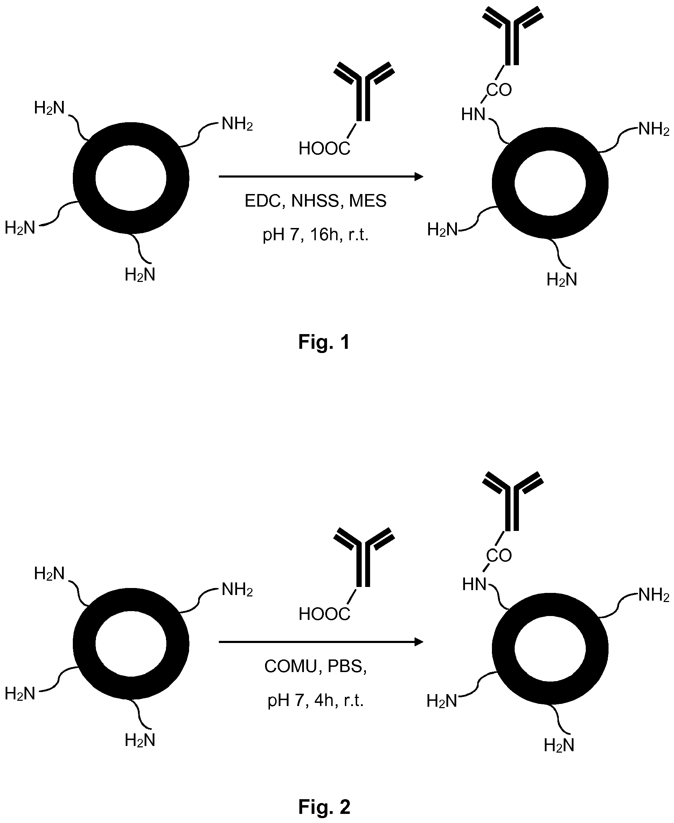

**Figure 1** schematically represents a prior art synthetic pathway to functionalize gold nanoparticles with Cetuximab antibodies;
**Figure 2** schematically represents a synthetic pathway according to aspects of the present invention to functionalize gold nanoparticles with Cetuximab antibodies;

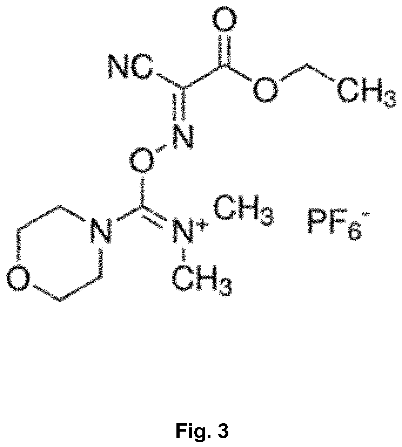

**Figure 3** schematically represents the molecular structure of COMU;

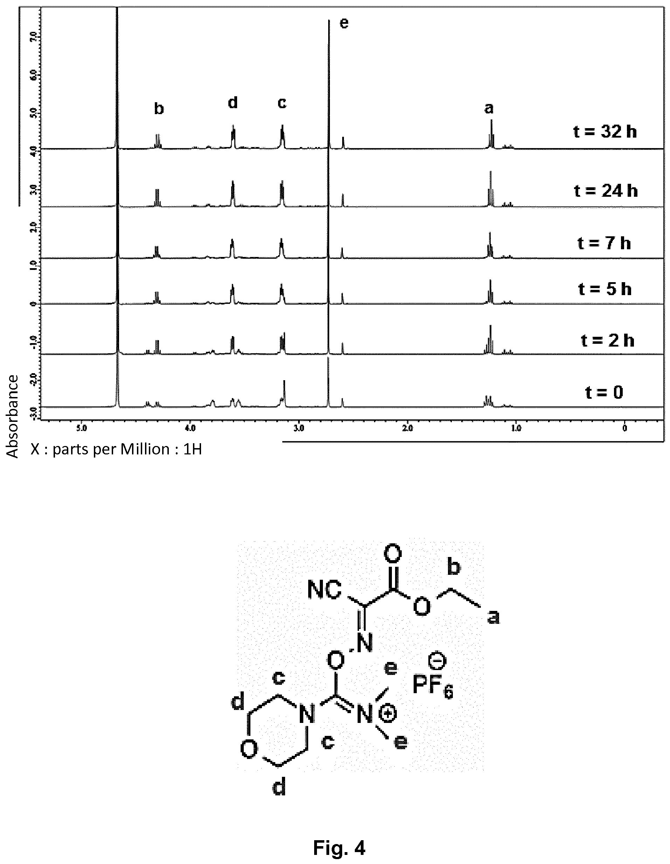

**Figure 4** reports the stability assessment of COMU in PBS, evaluated by ¹H-NMR in a time range comprised between dissolution of COMU in PBS (t=0) to 32h;

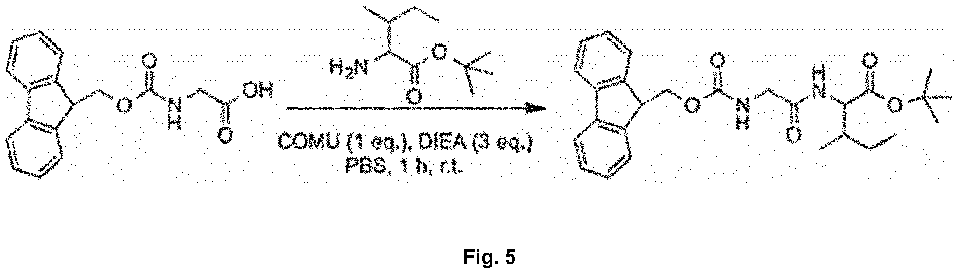

**Figure 5** schematically represents a synthetic pathway towards the formation of a dipeptide Fmoc-NH-Gly-Ile-OtBu in the presence of COMU as coupling agent and DIEA (N,N-Diisopropylethylamine) in PBS as solvent;

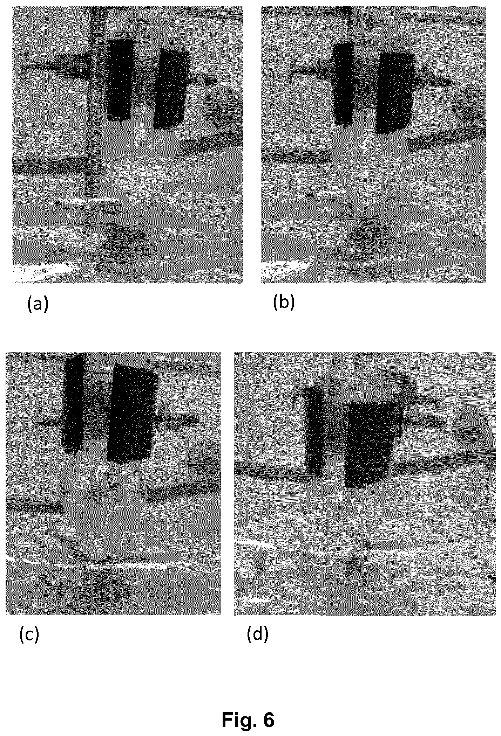

**Figure 6** shows optical photographs taken at different time intervals after the introduction of COMU into the reaction mixture described in Figure 5. The yellowish-coloured by-product is diagnostic of the occurred peptide coupling: (a) t=0 (i.e. before COMU addition); (b) t=1 min after COMU addition; (c) t=1 hour after COMU addition; (d) t=24 hours after COMU addition;

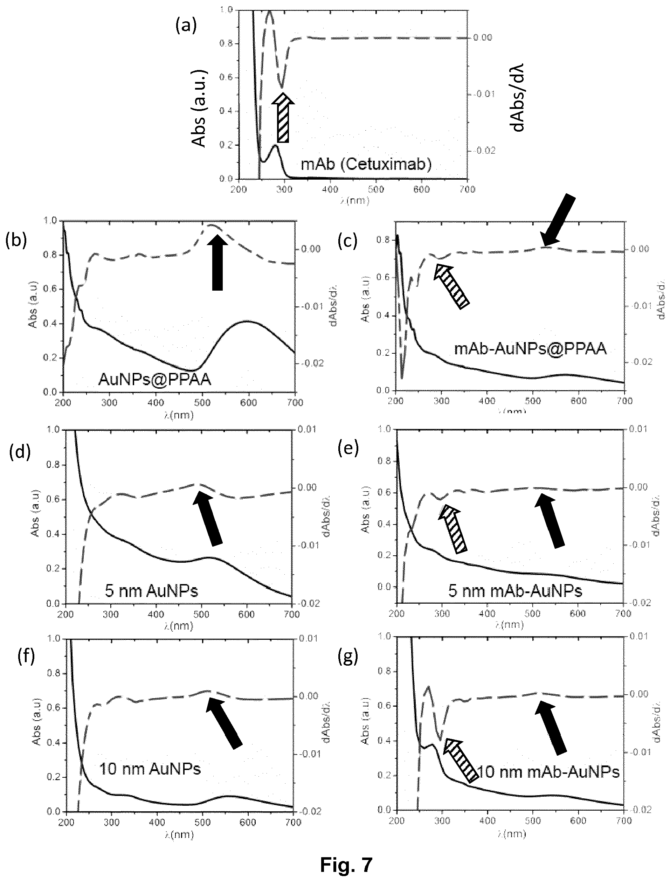

**Figure 7** shows steady-state UV-Visible absorbance profiles (full lines) and differential plots dAbs/dλ (dashed lines) of different materials before (**Figures 7a, 7b, 7d, 7f**) and after (**Figures 7c, 7e, 7g**) bioconjugation reaction with COMU for 16h: for Cetuximab (**Figure 7a**), AuNPs@PPAA (**Figure 7b**), Ctxb-AuNPs@PPAA (**Figure 7c**), commercial 5 nm AuNPs (**Figure 7d**), commercial 5 nm Ctxb-AuNPs (**Figure 7e**), 10 nm AuNPs (**Figure 7f**), and commercial 10 nm Ctxb-AuNPs (**Figure 7e**), with absorbance given in a.u. and wavelength λ in nm;

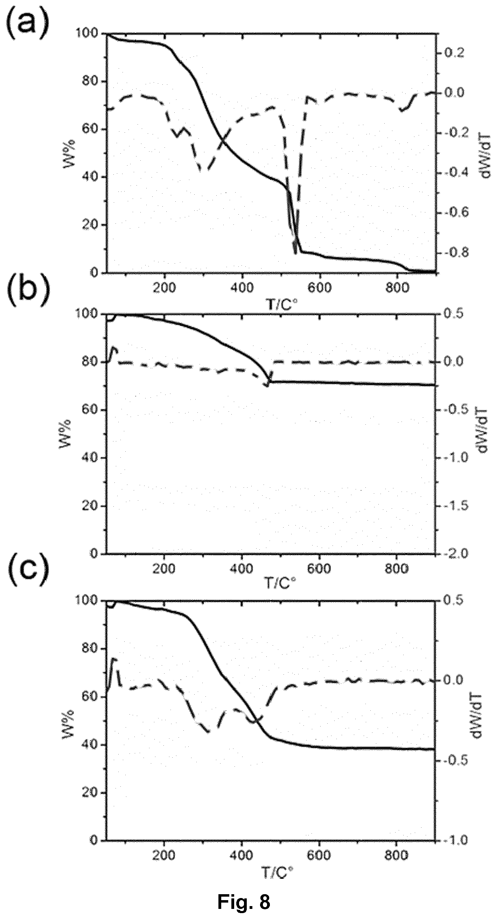

**Figure 8** shows thermogravimetric analysis (TGA) characterization of Ctxb-AuNPs@PPAA obtained after 16h of reaction with COMU: TGA of Cetuximab (**Figure 8a**), AuNPs@PPAA (**Figure 8b**) and Ctxb-AuNPs@PPAA (**Figure 8c**) with temperature-modulated plots (full lines) and differential plots dW/dT (dashed lines);

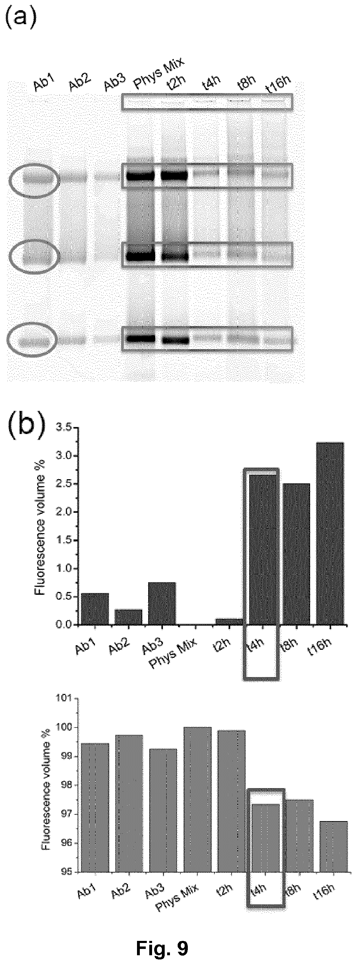

**Figure 9** shows fluorescence revelation (λ_{excitation} 488 nm, λₑₘᵢₛₛᵢₒₙ 500 nm) of polyacrylamide gel after electrophoresis of different aliquots pooled at different reaction times from the bioconjugation reaction mixture (**Figure 9a**); and fluorescence volume% repartition between the bands located in the deposition area and belonging to the free Cetuximab, as obtained by software editing of the gel revelation (top and bottom of **Figure 9b**, respectively);

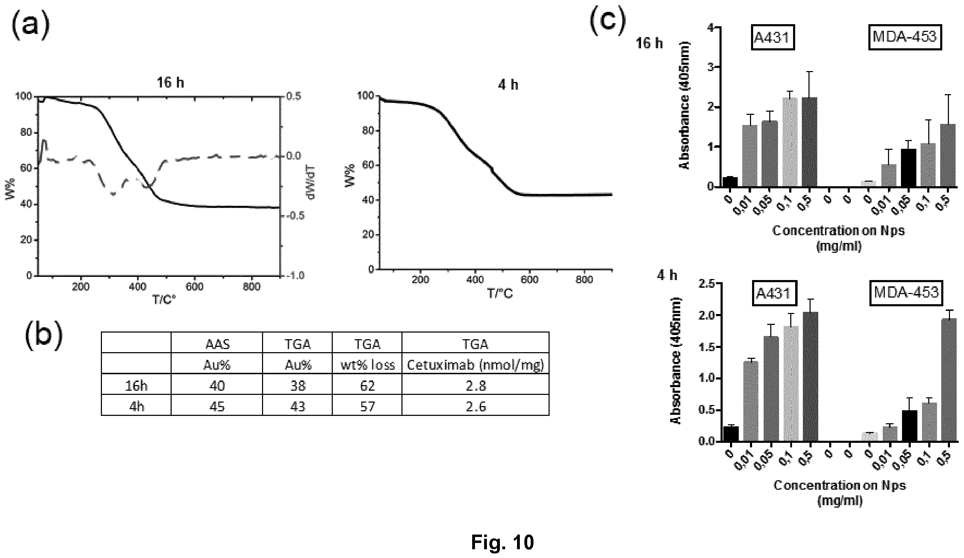

**Figure 10** shows a comparison of the (bio)chemical properties of Ctxb-AuNPs@PPAA bioconjugates obtained after 16h and 4h reaction time with COMU: TGA of Ctxb-AuNPs@PPAA (**Figure 10a**) with temperature-modulated plots (full lines) and differential plot (dashed lines); summary of TGA and atomic absorption spectroscopy (AAS) results (**Figure 10b**); and Enzyme-Linked Immunosorbent Assay (ELISA) on EGFR+ cells (A431) or EGFR- cells (MDA-MB-453) (**Figure 10c**);

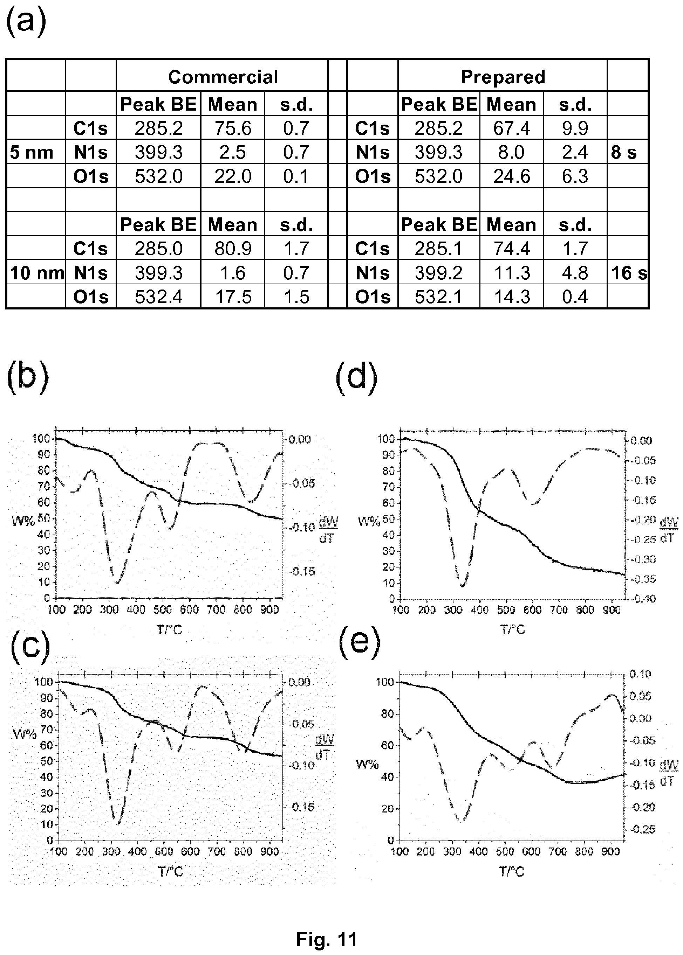

**Figure 11** shows a comparison of chemical composition of commercial available and prepared NH₂-bearing AuNPs as assessed by XPS analysis (**Figure 11a**). A TGA analysis before (**Figures 11b**, **11c**) and after (**Figures 11d, 11e**) the optimization of the bioconjugation reaction using commercial 5 nm (**Figures 11b**, **11d**) and 10 nm (**Figures 11c**, **11e**) AuNPs is shown, with temperature-modulated plots (full lines) and differential plots dW/dT (dashed lines).

Methods for functionalizing nanoparticles according to aspects of the present invention comprise the steps of:
- providing a first solution, said first solution comprising an organic molecule (or biological molecule) dissolved (or diluted) in phosphate buffered saline (or PBS), the organic molecule comprising a carboxyl group;
- providing a second solution, said second solution comprising 1-Cyano-2-ethoxy-2-oxoethylidenaminooxy)dimethylamino-morpholino-carbenium hexafluorophosphate (or COMU) dissolved (or diluted) in phosphate buffered saline (or PBS);
- adding the first solution to the second solution (so as) to form a third solution;
- stirring the third solution;
- adding nanoparticles to the third solution (so as) to form a fourth solution, the surface of said nanoparticles comprising amine functions;
- stirring the fourth solution.

In aspects of the present invention, the organic molecule comprises a carboxyl group. More particularly, the organic molecule comprises at least one carboxyl group. Otherwise stated, the organic molecule comprises one or more carboxyl group (i.e. the organic molecule comprises one, two, three, or more carboxyl groups).

Advantageously, the organic molecule (or biological molecule) is an antibody, a protein, carboxylated biotin, or carboxylated DNA, advantageously an antibody or a protein, advantageously an antibody.

More advantageously, the antibody is Cetuximab (throughout the description also referred to or denoted as Ctxb or mAb).

The molecular structure of COMU (C₁₂H₁₉F₆N₄O₄P, molecular weight 428.27 g/mol) is depicted in **Figure 3**.

Throughout the present description, COMU refers to 1-Cyano-2-ethoxy-2-oxoethylidenaminooxy)dimethylamino-morpholino-carbenium hexafluorophosphate.

COMU can also be referred to by its alternative names, i.e. as 1-[1-(Cyano-2-ethoxy-2-oxoethylideneaminooxy)-dimethylamino-morpholino]-uronium hexafluorophosphate, or 1-[(1-(cyano-2-ethoxy-2-oxoe-thylideneaminooxy)-dimethylaminomorpholinomethylene)] methanaminium hexafluorophosphate.

In other words, (the molecular structure of) COMU (depicted in **Figure 3**) can (interchangeably) be referred to by three names, i.e. 1-Cyano-2-ethoxy-2-oxoethylidenaminooxy)dimethylamino-morpholino-carbenium hexafluorophosphate, 1-[1-(Cyano-2-ethoxy-2-oxoethylideneaminooxy)-dimethylamino-morpholino]-uronium hexafluorophosphate, or 1-[(1-(cyano-2-ethoxy-2-oxoe-thylideneaminooxy)-dimethylamino-morpholinomethylene)] methanaminium hexafluorophosphate.

COMU is commercial available, and can for example be supplied by Iris Biotech GmbH or by Sigma-Aldrich.

Throughout the present description, phosphate buffered saline is denoted or referred to as PBS.

Advantageously, in aspects of the present invention, the step of stirring the third solution is performed at a temperature between 15°C and 40°C, advantageously between 15°C and 30°C, advantageously between 20°C and 25°C, advantageously at room temperature.

Advantageously, the step of stirring the third solution is performed for a time ranging between 10 and 30 minutes, advantageously between 10 and 20 minutes, advantageously for 15 minutes.

More advantageously, the step of stirring the third solution is performed at a temperature between 15°C and 40°C, advantageously between 15°C and 30°C, advantageously between 20°C and 25°C, advantageously at room temperature; and for a time ranging between 10 and 30 minutes, advantageously between 10 and 20 minutes, and advantageously for 15 minutes.

Advantageously, the step of stirring the fourth solution is performed at a temperature between 15°C and 40°C, advantageously between 15°C and 30°C, advantageously between 20°C and 25°C, advantageously at room temperature.

Advantageously, the step of stirring the fourth solution is performed for a time ranging between 2 and 8 hours, advantageously between 2 and 6 hours, advantageously between 3 and 5 hours, advantageously for 4 hours.

More advantageously, the step of stirring the fourth solution is performed at a temperature between 15°C and 40°C, advantageously between 15°C and 30°C, advantageously between 20°C and 25°C, advantageously at room temperature; and for a time ranging between 2 and 8 hours, advantageously between 2 and 6 hours, advantageously between 3 and 5 hours, advantageously for 4 hours.

In aspects of the present invention, COMU is used as coupling agent (or coupling additive, or coupling reagent) in PBS buffered solution for the functionalization of NH₂-bearing nanoparticles with organic molecules (comprising a carboxyl group) via the formation of an amide bond. In other words, aspects of the present invention involve the anchoring of organic molecules to amino-bearing nanoparticles via an amide bond by using COMU as coupling agent. Only one type of coupling agent is used.

In prior art methods for functionalizing nanoparticles, biological molecules are bound to metal nanoparticles based on:
i) carbodiimides and succinimidyl esters, such as EDC (1-ethyl-3-(3-dimethylaminopropyl)carbodiimide) and/or NHS (*N*-hydroxysuccinimide), using organic solvents, such as DMF (dimethylformamide) and MNP (2-methyl-2-nitrosopropane); or
ii) EDC/HOBt (hydroxybenzotriazole) in a buffer of MES (2-(N-morpholino)ethanesulfonic acid).
Hence, in the art, most of the time more than one type of coupling agent is used in the coupling reaction (i.e. most of the time two different coupling agents are used), leading to rather high reaction costs.

In **Figure 1**, an example of a prior art synthetic pathway for the preparation of bioconjugates between gold nanoparticles (AuNPs) and Cetuximab antibodies is schematically shown. The AuNPs used are coated with a layer of plasma-polymerized allylamine (PPAA), denoted as AuNPs@PPAA. The amino groups present in the polymeric shell of AuNPs@PPAA are exploited for amide bond formation with carboxyl groups located on Cetuximab, through carbodiimide chemistry using EDC, NHSS (N-hydroxysulfosuccinimide) as coupling agents and MES buffered solutions as solvent. Bioconjugates of antibodies coupled to the nanoparticles, Ctxb-AuNPs@PPAA, are thereby formed.

In the figures accompanying the present description, Cetuximab is represented by:

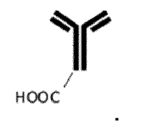

**Figure 1** describes an amide coupling reaction known in the art to produce antibody-functionalized gold nanoparticles. In particular, the chemical reaction involves first the activation of the Cetuximab carboxylic moieties by using EDC and NHSS followed by the subsequent amide bond formation in the presence of amine-terminating nanoparticles. The functionalization is performed in MES buffer (pH 7) at room temperature, for 16 hours.

**Figure 2** is a synthetic pathway for the synthesis of bioconjugates between gold nanoparticles (AuNPs) and Cetuximab antibodies according to an embodiment of aspects of the present invention. The AuNPs used are coated with a layer of plasma-polymerized allylamine (PPAA), denoted as AuNPs@PPAA. Contrary to the prior art methods, aspects of the present invention use COMU as one and only coupling agent with PBS as reaction media (or solvent). Bioconjugates of antibodies coupled to the nanoparticles, Ctxb-AuNPs@PPAA, are thereby formed.

In methods for functionalizing nanoparticles of aspects according to the invention, an organic molecule is covalently linked to the coupling agent COMU in PBS solution as reaction media (or solvent). More particularly, COMU activates the carboxylic moieties present on the organic molecule (for example on Cetuximab antibodies as depicted in **Figure 2**) prior to the amide coupling reaction towards the amine-terminating gold nanoparticles. Due to the high coupling efficiency of COMU together with its solubility and stability in phosphate ions-containing solutions (such as PBS buffered solution at pH 7), the bioconjugation in aspects of the invention is already completed after stirring the mixture for 2 to 8 hours, even after stirring for only 4 hours (at a temperature between 15°C and 40°C, advantageously at room temperature). In aspects of the present invention, no additional, separate steps of activating the nanoparticles and/or the organic molecule, are needed prior to performing the coupling reaction. Furthermore, the time for completing the coupling reaction is significantly reduced, compared to prior art methods. The number and amount of chemical reagents used is significantly reduced as well. Hence, methods of aspects of the invention are simplified, requiring less complex reactions (based on relative easy and simple chemical synthesis), and are performed under more simple reaction conditions. The methods of aspects of the invention are also faster and less costly.

It has been found that by using COMU in aspects of the present invention, the time for completing the coupling reaction between the organic molecule and the nanoparticles bearing amine-functions is significantly reduced (e.g. taking 2 to 8 hours, or even only 4 hours, using COMU in PBS solution instead of 16 hours by using prior art methods based on EDC chemistry and MES-based buffers, as will be further demonstrated in the examples of the present description). Hence, the coupling reaction is more efficient compared to prior art methods. Without being bound to theory, the reduction in time and the higher coupling efficiency is due to the presence of a morpholonium-based immonium moiety in the COMU coupling agent.

In methods for functionalizing nanoparticles of aspects according to the invention, it is thus proposed to use only one type of coupling agent, i.e. to only use COMU as coupling agent, whereas in the art, often two different types of coupling agents are used.

Furthermore, in the art, COMU is known to be highly stable and highly soluble in almost all organic solvents. Indeed, for amide coupling reactions in the art up to now, both in solution and solid phase, coupling agents (as for example COMU), are only being dissolved in organic solvents like dimethylformamide (DMF) and N-Methyl-2-pyrrolidone (NMP). In aspects of the present invention, it has been surprisingly found that COMU also has compatibility for reactions with PBS, i.e. COMU is not deactivated by PBS at all. More particularly, COMU unexpectedly appears to be stable and soluble in water and in (aqueous) PBS solution, contrary to most of the coupling agents used in prior art methods for functionalizing nanoparticles. Indeed, most coupling agents used in the art are not water soluble and will even be deactivated (degraded) by using PBS as solvent. For example, EDC is known to have short life times in PBS.

Hence, using COMU as one and only coupling agent in aspects of the invention allows the use of biocompatible reaction conditions using PBS as solvent (at room temperature (r.t.), with a coupling reaction of a few hours) instead of using organic solvents used in prior art methods for functionalizing nanoparticles. Moreover, COMU is non-explosive, due to the absence of a benzotriazole moiety, and has low toxicity, leading to a safer handling during the coupling reaction in aspects of the present invention. Hence, methods according to aspects of the present invention are less polluting and thus more environmentally friendly compared to prior art methods for nanoparticle functionalization, allowing the method to be compatible with a wide variety of biological applications.

The reaction mixtures formed by coupling reactions using COMU as coupling agent, according to aspects of the present invention, can thus be considered as being less toxic than the reaction mixtures formed by coupling reactions performed in the art using for example EDC and NHS. This can be beneficial for the formed (suspension of) functionalized nanoparticles obtained after purification, for their (its) further use in a variety of biological and medical applications, such as in cancer treatment.

In methods for functionalizing nanoparticles according to aspects of the present invention, the surface of the nanoparticles added to the third solution comprise amine functions (or NH₂-functions, or amino functionalities). In other words, the nanoparticles added to the third solution are amino-bearing (NH₂-bearing) nanoparticles. More particularly, the nanoparticles added to the third solution comprise a surface or shell or coating exohedrally exposing amine functions.

In the context of the present description, "functionalizing nanoparticles" refers to functionalizing the surface of nanoparticles with organic molecules (resulting in "functionalized nanoparticles"). More particularly, the amino-bearing surface of the nanoparticles is functionalized with organic molecules. In other words, bioconjugates (or conjugates) are formed between the (amine functions exposed on the surface of the) nanoparticles and the organic molecules.

Advantageously, in methods for functionalizing nanoparticles according to aspects of the present invention, the nanoparticles added to the third solution are metal nanoparticles or metal-based nanoparticles, the surface of said nanoparticles comprising amine functions.

More advantageously, the nanoparticles added to the third solution are metal nanoparticles, the surface of said nanoparticles comprising amine functions.

In the context of the present description, "metal nanoparticles" refer to nanoparticles comprising (or consisting of) a metal as such.

In the context of the present description, "metal-based nanoparticles" refer to nanoparticles comprising (or consisting of) metal alloys, metal oxides, metal nitrides, or metal carbides.

More advantageously, the metal of the metal nanoparticles or of the metal based-nanoparticles is selected from the group of transition metals, alkali metals, alkali earth metals, and lanthanides. More advantageously, the metal of the metal nanoparticles or of the metal-based nanoparticles is selected from the group comprising gold (Au), silver (Ag), platinum (Pt), palladium (Pd), iron (Fe), chromium (Cr), manganese (Mn), cobalt (Co), nickel (Ni), copper (Cu), silicon (Si), zirconium (Zr), yttrium (Y), indium (In), iridium (Ir), gallium (Ga), gadolinium (Gd), and titanium (Ti).

Even more advantageously, the metal of the metal nanoparticles is gold (or the metal nanoparticles are gold nanoparticles).

Advantageously, prior to the step of adding the nanoparticles to the third solution, bare (or naked) nanoparticles are coated with a layer of plasma-polymerized allylamine (an amine-functionalized polymer, denoted as PPAA). Advantageously, bare (or naked) metal nanoparticles, or bare (or naked) metal-based nanoparticles, are coated with a layer of PPAA.

Suitable methods for coating the nanoparticles with PPAA will be apparent for those skilled in the art. For example, polymer-coated AuNPs can be synthesized through step-wise and layer-by-layer plasma vapor deposition of Au (core) and plasma-polymerized allylamine (PPAA), leading to a shell exohedrally exposing amino functionalities, as described by N. Moreau et. Al. in Plasma Process. Polym., 2009, 6, S888.

More advantageously, the thickness of the layer of plasma-polymerized allylamine coated onto the bare (or naked) nanoparticles is lower than or equal to 50 nm.

Advantageously, before starting the bioconjugation reaction, (prepared) nanoparticles coated with a layer of plasma-polymerized allylamine are purified, and then isolated by lyophilisation. Advantageously, the purification is performed by discontinuous diafiltration against H₂O in centrifugal concentrators through regenerated cellulose membranes of 10,000 gmol⁻¹ as molecular weight cut-off (MWCO), or by tangential flow filtration with similar membranes in terms of MWCO.

Advantageously, before starting the bioconjugation reaction, (formulations of) the organic molecules comprising a carboxyl group (such as antibodies, proteins, carboxylated biotin, or carboxylated DNA), advantageously antibodies (e.g. Cetuximab or Cetuximab formulations), are purified, and then isolated by lyophilisation. Advantageously, the purification is performed by discontinuous diafiltration against H₂O in centrifugal concentrators through regenerated cellulose membranes of 5,000 or 10,000 gmol⁻¹ as molecular weight cut-off (MWCO), or by tangential flow filtration with similar membranes in terms of MWCO.

Advantageously, in methods for functionalizing nanoparticles according to aspects of the present invention, the shape of the nanoparticles added to the third solution comprises a sphere, a rod, a prism, a disk, a cube, a cage, a frame, or a mixture thereof. More advantageously, the shape of the nanoparticles added to the third solution is a sphere (or spherical nanoparticles are added to the third solution).

Advantageously, the nanoparticles added to the third solution have at least one dimension comprised between 1 nm and 100 nm.

More advantageously, the shape of the nanoparticles added to the third solution is a sphere and the diameter of the (bare or naked) nanoparticles is comprised between 1 nm and 100 nm, advantageously between 4 nm and 40 nm, advantageously between 4 nm and 30 nm, advantageously between 4 nm and 20 nm, advantageously between 4 nm and 10 nm.

Advantageously, in methods for functionalizing nanoparticles according to aspects of the present invention, the phosphate buffered saline has a molar concentration comprised between 5 mM and 0.5 M.

Advantageously, the phosphate buffered saline has a pH comprised between 6.4 and 7.6, advantageously between 6.6 and 7.5, advantageously between 6.8 and 7.4.

Advantageously, the mass concentration of organic molecule in the first solution is comprised between 0.5 mgmL⁻¹ and 2.0 mgmL⁻¹.

Advantageously, the mass concentration of COMU in the second solution is comprised between 0.5 mgmL⁻¹ and 2.5 mgmL⁻¹.

Advantageously, the COMU to organic molecule weight ratio in the third solution is comprised between 0.5:1 to 2:1.

Advantageously, the COMU to organic molecule molar ratio in the third solution is comprised between 177:1 to 710:1.

Advantageously, the nanoparticles are added to the third solution in an amount comprised between 0.2 and 2 mg nanoparticles per mg organic molecule.

Advantageously, the pH in the fourth solution is comprised between 6.0 and 8.0, advantageously between 6.6 and 7.4, advantageously between 6.8 and 7.2, advantageously the pH in the fourth solution is 7.0.

Advantageously, performing the step of stirring the fourth solution yields a colloidal suspension of nanoparticles functionalized with organic molecules, advantageously with antibodies, i.e. bioconjugates are formed between the nanoparticles and the organic molecules, advantageously the antibodies.

In other words, performing methods for functionalizing nanoparticles according to aspects of the present invention is yielding a colloidal suspension of nanoparticles (surface) functionalized (or bioconjugated) with organic molecules, advantageously with antibodies.

Advantageously, in methods for functionalizing nanoparticles according to aspects of the present invention, after the step of stirring the fourth solution, the solution is purified so as to obtain purified (surface) functionalized (or bioconjugated) nanoparticles in suspension. More particularly, by performing the step of purification a purified colloidal suspension of functionalized (or bioconjugated) nanoparticles is obtained.

More advantageously, purification of the fourth solution is performed by discontinuous diafiltration against H₂O in centrifugal concentrators (with MWCO of 300,000 g/mol) or by tangential flow filtration with similar membranes in terms of MWCO.

In the context of the present description, molecular weight cut-off (or MWCO) refers to the lowest molecular weight solute (in Daltons) in which 90% of the solute is retained by the used membrane.

More advantageously, the purified functionalized (or bioconjugated) nanoparticles are isolated from the suspension by lyophilization (or freeze-drying), or the suspension is supplemented with Arabic gum prior to lyophilization.

According to an embodiment of aspects of the invention, a first solution is provided, said first solution comprising antibody Cetuximab dissolved (or diluted) in PBS; a second solution is provided, said second solution comprising COMU dissolved (or diluted) in PBS; the first solution is added to the second solution (so as) to form a third solution; the third solution is stirred at room temperature for 15 minutes; nanoparticles, advantageously gold nanoparticles, are added to the third solution (so as) to form a fourth solution, the surface of said nanoparticles comprising amine functions; the fourth solution is stirred at room temperature for 4 hours. In this embodiment, COMU is used as coupling agent in PBS buffered solution for the functionalization of NH₂-bearing nanoparticles, advantageously gold nanoparticles with Cetuximab antibodies via an amide bond.

According to another aspect of the present invention, there is provided a colloidal suspension comprising functionalized nanoparticles (or colloidal nanoparticle suspension) obtainable by methods according to the invention.

According to yet another aspect, the present invention provides functionalized nanoparticles obtainable by methods according to the invention.

According to still another aspect, the present invention provides functionalized nanoparticles, or a colloidal suspension comprising functionalized nanoparticles, obtainable by methods according to the invention for use in therapy (for use in treatment by therapy) or for use in *in vivo* diagnostics (for use in *in vivo* diagnostic methods), advantageously for use in a method for treatment of cancer or for use in a method of diagnosis *in vivo* of cancer

Indeed, functionalized nanoparticles, or a colloidal suspension comprising functionalized nanoparticles, obtainable by methods according to aspects of the invention can be employed in a wide variety of medical and biological applications. The obtained (suspension of) nanoparticles can be used *inter alia* as sensor, in imaging, or in drug-delivery. The formed (suspension of) nanoparticles can as well be applied in cancer targeting *in vivo* or in cancer treatment.

The resulting (suspension of) nanoparticles functionalized with appropriate biological molecules, advantageously antibodies, can for example also be used in order to enhance the efficiency of radio and proton therapy for improving cancer treatment. Furthermore, such functionalized nanoparticles can allow for targeting *in vivo* localization of the nanoparticles, for directing the nanoparticles to specific sites within a body, or for following the movement of specific proteins or RNA molecules in living cells.

More particularly, antibodies targeting for example the epidermal growth factor receptor (EGFR), a membrane protein overexpressed in several kinds of solid tumours, can be conjugated to gold nanoparticles bearing amine-functions and the resulting bioconjugates can be efficiently employed to assess EGFR targeting *in vivo,* for imaging photothermal treatment, and for drug delivery.

As such, antibody-conjugated nanoparticles obtainable by methods according to aspects of the invention can offer increased selectivity and sensitivity in the diagnosis of cancer using imaging techniques such as magnetic resonance imaging (MRI), positron emission tomography (PET), and computed tomography (CT).

In aspects of the invention, the organic molecules (or biological molecules, or biomolecules), advantageously antibodies, proteins, carboxylated biotin, or carboxylated DNA, advantageously antibodies or proteins, advantageously antibodies, can be radiolabelled with therapeutic markers (or radio-isotopes, radio-nuclides, or radio-emitters). The biomolecules can for example be radioiodinated with ¹²⁵I or presenting a chelate of ⁸⁹Zr. Alternatively, the biomolecules can be tagged with fluorescent dyes (i.e. cyanine-based derivatives).

Suitable methods for radiolabelling, the organic molecules will be apparent for those skilled in the art.

Radioiodination (for instance of antibodies) can for example be performed using the lodo-Gen kit from Pierce Thermo Scientific (using the Thermo Scientific Pierce^{®} Iodination Reagent, see e.g. R. Marega et. al. in J. Mater. Chem. 2012, 22, 21305). ⁸⁹Zr radiolabelling (of for instance antibodies) can for example be performed by reacting Ctxb according to the method reported in the literature (see e.g. L. Karmani, Nanomedicine 2014, 9, 1923).

### EXAMPLES

In the context of the present description, wt% (or % (w/w)) refers to the percentage of the component under consideration by total weight of the structure under consideration.

Throughout the present examples, commercial available COMU from Iris Biotech GmbH has been used.

### A. Solubility and stability of COMU in PBS and COMU efficiency

The solubility of COMU in PBS has quantitatively been assessed by standard solubilization assays (increasing the concentration of the reagent while the solvent volume is maintaining constant), indicating a maximum product solubility of 4 mg/mL (around 9 mM). Assessing the solubility by standard solubilization assays is apparent for those skilled in the art.

The stability of COMU in PBS has been monitored upon time by ¹H-NMR studies. ¹H-NMR spectra are recorded during a time (t) comprised between dissolution of COMU in PBS (t=0) and 32 hours. The resulting diagram is represented in Figure 4, and showing that the phosphate ions do not react with the carbodiimide-like active core, even not after 32h, which is the double of the time for the bioconjugation reaction used in methods known in the prior art.

COMU efficiency in mediating amide bond formation in PBS media (or solvent) was tested through a model reaction involving two amino acids, Fmoc-NH-Gly-OH and H₂N-Ile-OtBu, to form the dipeptide Fmoc-NH-Gly-Ile-OtBu. The synthetic pathway is shown in **Figure 5**.

Optical photographs taken at different time intervals after the introduction of COMU into a reaction mixture are depicted in **Figure 6**: (a) t=0 (i.e. before COMU addition); (b) t=1 min after COMU addition; (c) t=1 hour after COMU addition; (d) t=24 hours after COMU addition. Since the COMU by-product is a chromophore in the visible range, a time-dependent increase of a yellowish coloration can visually be seen, attesting the ongoing increase of amide bonds formed. Colour changes during the coupling reaction hence allow visual or colorimetric reaction monitoring in aspects of the present invention.

### B. Characterization of formed nanoparticle bioconjugates

A set of bioconjugation reactions was performed in order to obtain sufficient amounts of material to perform the characterization of both the structural and morphological features of the produced nanoparticle bioconjugates.

As starting material for the nanoparticles, either commercial available (i.e. from AC Diagnostics, Inc.) or prepared NH₂-bearing gold nanoparticles were used. The prepared NH₂-bearing gold nanoparticles were obtained through step-wise and layer-by-layer plasma vapor deposition of Au (core) and plasma-polymerized allylamine, as described by N. Moreau et. Al. in Plasma Process. Polym., 2009, 6, S888, forming gold nanoparticles comprising a coating exohedrally exposing amine functions (denoted as AuNPs@PPAA).

As starting material for organic molecules to conjugate to the nanoparticles, commercially-available anti-EGFR antibodies were used. More particularly, antibody Cetuximab (Ctxb) formulation, being commercially available as Erbitux^{®} 2 or 5 mg mL⁻¹ solutions of 20 to 100 mL by Merck & Co, was used.

Before starting the bioconjugation reactions, Cetuximab formulations and prepared NH₂-bearing gold nanoparticles were first purified by size-separation techniques (i.e. centrifugal concentrators or tangential flow filtration systems), and then isolated by lyophilisation. As separation elements of the size-separation systems, both polyetheresulphone membranes (molecular weight cut-off (MWCO) of 5,000 gmol-1 or 30,000 gmol⁻¹) or regenerated cellulose (RC) membranes (10,000 gmol⁻¹ MWCO) were used, with similar efficiency in terms of purification time and product recovery. For the commercial available nanoparticles, only the lyophilisation step was performed (no purification is needed), before starting the bioconjugation reactions.

Initially, the bioconjugations were performed with a reaction time of 16 hours, by employing discontinuous diafiltration against H₂O (MWCO 300,000 gmol⁻¹) for product purification and subsequent isolation through lyophilisation. This procedure yielded bioconjugated materials (Ctxb-AuNPs@PPAA) at the 5 mg scale, with yields of w/w of around 40 to 70% considering the employed AuNPs mass, starting from the prepared NH₂-bearing gold nanoparticles. By using the commercial AuNPs, the bioconjugation reaction is much less efficient, i.e. 5 nm and 10 nm Ctxb-AuNPs, with yields of w/w around of 10 to 30%. This is probably due to material loss inside the polyetheresulphone (PES) membrane of the diafiltration tubes.

The obtained bioconjugated materials were initially analysed by UV-visible spectroscopy, aiming at identifying the changes in the AuNPs upon the introduction of the peptide moiety of Cetuximab. This qualitative assessment is based on the different UV-visible profile of the starting materials (i.e. plasmonic Au peak at around 520 nm for the AuNPs, and aromatic amino acids absorption at 280 nm for Cetuximab), and on the possibility of removing the physisorbed Cetuximab from the AuNPs upon the diafiltration process. Therefore, the contemporaneous presence of both of the aforementioned absorption features in all sets of bioconjugates represents an initial evidence that the AuNPs bioconjugation occurred.

**Figure 7** shows the steady-state UV-Visible absorbance profiles (full lines) and differential plots dAbs/dλ (dashed lines) of different materials before and after bioconjugation reaction with COMU for 16h. The AuNPs plasmonic peak and the position of the aromatic amino acid absorbance of the protein moiety are indicated by black arrows and dashed arrows, respectively.

Since Ctxb-AuNPs@PPAA was always isolated with higher yields, it was used as the material to perform the subsequent characterization steps of the compositional assessments (by thermogravimetric analysis (TGA) and atomic absorption spectroscopy (AAS)) and size distribution assessments (by CPS Disc Centrifuge from CPS Instruments).

Thermogravimetric analysis (TGA) is a pivotal characterization technique for hybrid organic-inorganic nanomaterials, being a bulk assessment of solids (on the sub-mg scale) that allows for the discrimination of the weight losses due to residual solvent or moisture (in the range of 50°C to 100°C), organic moiety (in the range of 100°C to 600 °C) and for the determination of the residual weight after the thermal scan due to the inorganic part (see for example E. Mansfield et. al. in Anal. Chem. 2014, 86, 1478). Therefore, TGA represents an ideal characterization technique for the precise assessment of AuNPs bioconjugates composition, which will lead to more accurate product handling for all the subsequent biological investigations where the exact quantification of the biologically active moieties (Ctxb and Au amount) is mandatory for data comparison and rationalization.

**Figure 8** shows the TGA characterization of Ctxb-AuNPs@PPAA obtained after 16h of reaction with COMU, i.e. TGA of Cetuximab (**Figure 8a**), AuNPs@PPAA (**Figure 8b**) and Ctxb-AuNPs@PPAA (**Figure 8c**) are reported as temperature-modulated plots (full lines) and differential plots dW/dT (dashed lines). The TGA of Cetuximab depicted in **Figure 8a** shows a decomposition occurring in the range between 100°C and 800 °C, with a residual weight at 950 °C close to 0. AuNPs@PPAA materials typically shows decomposition between 100°C and 500°C, as depicted in **Figure 8b**, due to the polyallylamine moiety (around 30 wt%, thus approximately 5.0 µmol allylamine mg⁻¹ of AuNPs@PPAA). Ctxb-AuNPs@PPAA TGA plots, as depicted in **Figure 8c**, clearly indicate an augmented weight loss (around 60wt%), which is thus due to the Cetuximab introduction.

It is hence possible to estimate the Ctxb-AuNPs composition as being 40 wt% Au, 17 wt% polyallylamine, and 43 wt% Cetuximab (2.8 nmol Cetuximab/mg of Ctxb-AuNPs@PPAA). This initial results attested the feasibility of substituting the carbodiimide-mediated procedure with the use of COMU and PBS as reagent and medium according to aspects of the present invention.

### C. Reduction of reaction time

Based on the above results, the possibility of reducing the reaction time (i.e. less than 16 hours) was screened, aiming at further simplifying and optimizing the reaction conditions and subsequent possible scale-up to industrial level. Therefore, a procedure to assess the possible time-dependency of Ctxb irreversible immobilisation on to AuNPs@PPAA was developed. After pilot experiments aimed at assessing AuNPs@PPAA interaction with polyacrylamide gels, fluorescence revelation of dye tagged Ctxb-AuNPs@PPAA was adopted as the method for such time-dependent screening. This was based on the fact that Ctxb-AuNPs@PPAA bioconjugated material cannot enter inside the small pores of a polyacrylamide gel (4 to 12% of reticulation), hence remaining confined in the deposition spot, while loosely bound or excess Cetuximab in the reaction media can migrate inside such gel.

Therefore, the following procedure was developed:
i) Pooling of bioconjugation reaction aliquots at different time intervals after COMU addition (t=0h, 2h, 8h, and 16h);
ii) Aliquots diafiltration through PES membranes with 10,000 gmol⁻¹ MWCO to remove the exceeding COMU, the reaction by-products and desalting;
iii) Diafiltered aliquots reaction with Cy2-NHS, an amino-group reactive dye with green fluorescence (λ_{excitation} = 488 nm), and purification;
iv) Gel electrophoresis and fluorescence scan.

The results of this assessments are reported in **Figure 9**. The fluorescence scan of the control lanes (lanes 1 to 3, loaded with Cetuximab at three different, decreasing amounts) clearly shows lack of Cy2 fluorescence in the deposition area, and three major bands corresponding to integral Cetuximab, and heavy and light chains fragmentation (**Figure 9a**). Lanes 4 to 8 were loaded with the different aliquots isolated at different times, which show very light fluorescence at the deposition area of the physical mixture (reaction at t=0), and increasing intensity upon increased reaction times (2h, 4h, 8h, and 16h). The fluorescence intensity of the bands corresponding to free Cetuximab seems to follow the opposite trend, being maximized at t=0 and t=2h, and almost identical after such time point. Furthermore, from the fluorescence volume at the deposition area and the fluorescence volume of free Cetuximab represented in top and bottom of **Figure 9b**, respectively, it clearly emerges that t=4h is already a good reaction time in the presence of COMU to obtain the highest fluorescence intensity at the deposition area and the lowest for the free Cetuximab bands. The time for performing the coupling reaction in methods according to aspects of the present invention is thus substantially reduced, compared to the prior art methods for functionalizing nanoparticles

### D. Biochemical investigation

A set of bioconjugation reactions was performed to obtain sufficient material to perform the aforementioned compositional and size distribution assessment, along with a biochemical investigation about the specific binding toward the selected biological target (EGFR overexpressing cells).

TGA traces of Ctxb-AuNPs@PPAA show similar weight loss (around 60 wt%), resulting in comparable Cetuximab loadings and Au wt% (40 and 45 wt%), confirmed by atomic absorption spectroscopy (AAS), as shown in **Figure 10a** and **Figure 10b**.

Once interfaced with either A431 cells (model of epidermoid carcinoma cells, overexpressing the EGFR) or MDA-MB-453 (cells lacking of significant EGFR expression) in a cell based Enzyme-Linked Immunosorbent Assay (ELISA, by Sigma-Aldrich), Ctxb-AuNPs@PPAA showed a similar dose-dependent binding (range 0.01 to 0.5 mg/mL of total material, around 0.004 to 0.2 mg of Au), regardless of the overall reaction time, as shown in **Figure 10c**.

Being Ctxb-AuNPs@PPAA, a nanostructured material with both positive and negative charges due to the Cetuximab moiety, the presence of a non-negligible aspecific binding toward the EGFR negative (EGFR-) cells is in line with earlier literature reports (e.g. R. Marega et. al. in J. Mater. Chem. 2012, 22, 21305; R. Marega in Adv. Funct. Mater. 2013, 23, 3173) dealing with other models of EGFR- cells, such as EAhy926 and CHO.

### E. Comparison between commercial AuNPs and prepared AuNPs

The above optimized reaction conditions were then applied to commercial available AuNPs (i.e. from AC Diagnostics, Inc.).

Besides the size distribution properties, commercial available AuNPs differ from the prepared AuNPs (i.e. prepared as in section B above), especially in terms of chemical composition.

Indeed, commercial available AuNPs have a chemisorbed dihydrolipoic acid (DHLA) moiety which is much different than the physisorbed PPAA present in the prepared AuNPs in terms of:
i) Au surface coverage (expressed in terms of molecules nm⁻²):
   Short thiols, such as mercapto-undecanoic acid, are densely packed (up to 9 nm⁻²) on commercial available AuNPs while thiolated polyethylene glycol derivatives range between 0.2 to 1 nm⁻², according to the molecular weight (cf. e.g. H. Hinterwirth et. al. in ACS Nano 2013, 7, 1129; X. Xia et. al. in ACS Nano 2012, 6, 512). It should be pointed out that plasma-polymerized materials can easily chemisorb on Au by exceeding the monolayer structure, and thus exceed the aforementioned densities. Furthermore, one way to express the polymer molecular weight is based on the weight of its monomeric constituent (about 60 gmol⁻¹ for allylamine, which is 10 times less than the DHLA moiety molecular weight);
ii) Organic-to-inorganic moieties ratio (expressed as µmol per mg of material):
   The generation of (sub)monolayers after DHLA chemisorption, or the PPAA reticulation around AuNPs, determines the presence of the different organic shells, exposing either allylamine units or amino-ethyleneoxy-groups, both amenable for the reaction with the carboxylates located on Cetuximab.

To detect the differences in the chemical composition of the AuNPs (i.e. of commercial vs. prepared AuNPs@PPAA), X-ray photoelectron spectroscopy (XPS) analysis and TGA were performed. XPS revealed similar compositions among commercial AuNPs (5 nm vs. 10 nm) and prepared AuNPs@PPAA (8s vs. 16s). However, some remarkable differences do exist between the two groups of materials. More particularly, commercial AuNPs show an abridged amount of nitrogen atoms (1.6 < at% < 2.6), compared to the amount found in AuNPs@PPAA (8.0 < at% < 11.3), as can be seen from **Figure 11a**. Furthermore, TGA reveals an overall higher amount of organic moiety for the commercial AuNPs (around 45 wt%, cf. graphs in the left part of **Figure 11b** and **Figure 11c**) compared to the amount detected in the prepared AuNPs@PPAA (25-30 wt%, cf. **Figure 8b**). These differences revealed by XPS and TGA well match with the structural properties of the organic shells (PEG and PPAA) surrounding the different AuNPs. Also for this reason, the corresponding Ctxb-bioconjugates show different degrees of Ctxb loading, as seen by the weight loss values resulting from TGA of the commercial bioconjugates (around 90 wt% for 5 nm commercial AuNPs, and around 60 wt% for 10 nm commercial AuNPs, right traces in **Figure 11b** and **Figure 11c**, respectively), and that of the prepared AuNPs@PPAA bioconjugates (Ctxb-AuNPs@PPAA, around 45 wt%, cf. **Figure 8c**).

**Table 1** summarizes all the physicochemical properties of representative samples for both commercial available and prepared AuNPs before and after the bioconjugation with Cetuximab, based on the above results obtained from XPS, TGA and fluorescamine assay.

**Table 1: Summary of physicochemical properties of commercial and prepared AuNPs, and the related Ctxb bioconjugates obtained by using COMU.**

| | **Commercial AuNPs** | | **Prepared AuNPs** | |
|---|---|---|---|---|
| | **5 nm** | **10 nm** | **8 s** | **16 s** |
| Au diameter (nm) | 4-10 (5) | 10-30 (14) | 5-7 (6) | 4-20 (5) |
| Au concentration (ppm) | 78 | 78 | 140 | 340 |
| Au concentration (mg mL⁻¹) | 0.078 | 0.078 | 0.14 | 0.34 |
| N° AuNP mL⁻¹ | 3.8x10¹³ | 5.6x10¹² | 7.7x10¹⁴ | 5.89x10¹³ |
| NH₂ loading by TGA (µmol mg⁻¹) | 0.7 | 0.7 | 4 | 5 |
| N° R-NH₂ AuNP⁻¹ (size distribution mean in bold) | 5.3x10¹ (4 nm) | 8.3x10³ (10 nm) | 4.4x10² (5 nm) | 2.9x10² (4 nm) |
| | **1.0x10² (5 nm)** | **2.3x10³ (14 nm)** | **7.7x10² (6 nm)** | **5.7x10² (5 nm)** |
| | 8.3x10³ (10 nm) | 2.2x10⁴ (30 nm) | 1.2x10³ (7 nm) | 3.7x10⁴ (20 nm) |
| N° R-NH₂ nm⁻² | 1 | 1 | 7 | 7 |
| Reactive NH₂ groups by fluorescamine assay (µmol mg⁻¹) | 0.42 ± 0.006 | 0.25 ± 0.028 | - | - |
| Surface composition (at%) (mean values of main peak binding energy (BE), XPS) | C: 75.6 ± 0.7 | C: 80.9 ± 1.7 | C: 67.4 ± 9.9 | C: 74.4 ± 1.7 |
| | N: 2.5 ± 0.7 | N: 1.6 ± 0.7 | N: 8.0 ± 2.4 | N: 11.3 ± 4.8 |
| | O: 22.0 ± 0.1 | O: 17.5 ± 0.1 | O: 24.6 ± 6.3 | O: 14.3 ± 0.4 |
| Moisture (%) | 1-10% | | | |
| Reaction yield (wt%) | 10-20 | 20-30 | 50-70 | 50-70 |
| mAb loading (Cetuximab) µmol mg⁻¹ | 5 | 2 | 4 | 3 |

### F. Bioconjugation protocol (general)

- Antibody (e.g. Cetuximab) is obtained from a commercial source (e.g. for Cetuximab, Erbitux^{®} 2 or 5 mg mL⁻¹ solutions of 20 to 100 mL by Merck & Co) or results from a process of radiolabelling (for example, radioiodination of antibodies can be performed using the lodo-Gen kit from Pierce Thermo Scientific, using the Thermo Scientific Pierce^{®} Iodination Reagent); the antibody is either dissolved or diluted in phosphate buffer saline (PBS, with molarity in the range 5 mM - 0.5 M and pH between 6.6 and 7.4) to final concentrations ranging between 0.5 - 2.0 mgmL⁻¹ (solution 1);
- COMU is dissolved in phosphate buffer saline (PBS, with molarity in the range 5 mM - 0.5 M and pH between 6.6 and 7.4) to final concentrations ranging between 0.5 - 2.5 mgmL⁻¹ (solution 2);
- solution 3 is obtained by adding solution 1 to solution 2 with COMU to antibody (e.g. Cetuximab) weight ratios between 0.5:1 to 2:1 (molar ratios ranging from 177:1 to 710:1, respectively), and is kept under stirring at room temperature for 15 minutes;
- AuNPs, either prepared (as set out in section B above) or from a commercial source (from AC Diagnostics, Inc.) and with Au amounts ranging between 0.2 and 2 mg Au/mg antibody (e.g. Cetuximab), are added to solution 3, yielding solution 4 (pH around 7);
- solution 4 is stirred at room temperature for a time ranging between 2 and 8 hours, after which it is purified by either discontinuous diafiltration against H₂O in centrifugal concentrators or by tangential flow filtration. The purified material can be maintained in suspension, lyophilized or supplemented with Arabic gum prior to lyophilization.

Using organic molecules comprising one or more carboxyl group (e.g. antibody Cetuximab), the carboxylic moiety of the organic molecule (e.g. of Cetuximab) does not require any additional or separate activation prior to the coupling reaction, due to the high coupling efficiency of COMU together with its solubility in PBS buffered solution.

### G. Bioconjugation protocol (specific)

- Erbitux formulation (Erbitux^{®} 2 or 5 mgmL⁻¹ solutions of 20 to 100 mL by Merck & Co) is purified by discontinuous diafiltration against water in centrifugal concentrators of 5,000 gmol⁻¹, yielding Cetuximab (Ctxb);
- Ctxb (5 mg) is dissolved in 5 mL of 0.067 M PBS buffer, and then a solution of COMU (from Iris Biotech GmbH; 9 mg, 0.02 mmol) in 6 mL of 0.067 M PBS buffer is added;
- the resulting solution is stirred at 25 °C for 15 min; and after that
- a solution of prepared AuNPs (prepared as set out in section B above), 3.6 mg Au in 5 mL of 0.067 M PBS buffer, is added;
- the solution is stirred at 25 °C for 4 hours, after which it is purified by discontinuous diafiltration against H₂O in centrifugal concentrators with MWCO of 300,000 g mol⁻¹;
- the product is isolated through freeze-drying, obtaining a pinkish red powder.

### H. Cost estimation

Using COMU (from Iris Biotech GmbH, 25g, 125 €) and PBS (from Fisher, 500 mL, 18 €) in aspects of the present invention, the cost to bioconjugate 5 mg of nanoparticles is 80.5 cents, meaning 16.1 cents per mg/nanoparticle. To the contrary, using EDC chemistry: HCm (1g 19.90 €), NHSS (5g 18.00 €) and MES (25g 47.90 €), the cost for 5 mg of nanoparticles is 410 cents, meaning 82 cents per mg/nanoparticle. The cost for performing methods according to aspects of the present invention is thus substantially reduced, compared to the prior art methods for functionalizing nanoparticles.

Even though in the above examples methods for functionalizing nanoparticles according to aspects of the invention are illustrated using antibody Cetuximab as organic molecule to functionalize gold nanoparticles, it will be clear to those skilled in the art that also other types of organic molecules, as well as other types of nanoparticles, can similarly be used in aspects of the invention.

From the description and examples above, it follows that the present invention thus provides an optimization of the bioconjugation reaction between organic molecules and nanoparticles, both in terms of efficiency and chemical reagents involved, compared with known methods in the art. More particularly, methods of aspects of the invention allow a faster and more efficient functionalization of nanoparticles presenting amine functions with organic molecules comprising a carboxyl group (such as antibodies, proteins, carboxylated biotin, or carboxylated DNA) using non-explosive and less toxic chemical compounds. Methods of aspects of the invention provide the functionalization of nanoparticles performed under simpler reaction conditions, reducing the number of reagents and reaction steps. As a consequence, methods of aspects of the invention do not require high costs. Moreover, the methods are based on relative easy and simple chemical synthesis and the followed purification is easy to perform as well, hence allowing the production of the bioconjugates nanoparticles to scale up to industrial level.

## Claims

1. Method for functionalizing nanoparticles, said method comprising the steps of:
- providing a first solution, said first solution comprising an organic molecule dissolved in phosphate buffered saline, the organic molecule comprising a carboxyl group;
- providing a second solution, said second solution comprising 1-Cyano-2-ethoxy-2-oxoethylidenaminooxy)dimethylamino-morpholino-carbenium hexafluorophosphate dissolved in phosphate buffered saline;
- adding the first solution to the second solution to form a third solution;
- stirring the third solution;
- adding nanoparticles to the third solution to form a fourth solution, the surface of said nanoparticles comprising amine functions;
- stirring the fourth solution.

2. Method of claim 1, wherein the step of stirring the third solution is performed at a temperature between 15°C and 40°C, and for a time ranging between 10 and 30 minutes.

3. Method of claim 1 or 2, wherein the step of stirring the fourth solution is performed at a temperature between 15°C and 40°C, and for a time ranging between 2 and 8 hours.

4. Method of any one of the preceding claims, wherein the organic molecule is an antibody, a protein, carboxylated biotin, or carboxylated DNA.

5. Method of any one of the preceding claims, wherein the antibody is Cetuximab.

6. Method of any one of the preceding claims, wherein the nanoparticles added to the third solution are metal nanoparticles or metal-based nanoparticles, the surface of said nanoparticles comprising amine functions.

7. Method of any one of the preceding claims, wherein the phosphate buffered saline has a molar concentration comprised between 5 mM and 0.5 M.

8. Method of any one of the preceding claims, wherein the mass concentration of organic molecule in the first solution is comprised between 0.5 mgmL⁻¹ and 2.0 mgmL⁻¹.

9. Method of any one of the preceding claims, wherein the mass concentration of COMU in the second solution is comprised between 0.5 mgmL⁻¹ and 2.5 mgmL⁻¹.

10. Method of any one of the preceding claims, wherein the COMU to organic molecule weight ratio in the third solution is comprised between 0.5:1 to 2:1.

11. Method of any one of the preceding claims, wherein the nanoparticles are added to the third solution in an amount comprised between 0.2 and 2 mg nanoparticles per mg organic molecule.

12. Method of any one of the preceding claims, wherein after the step of stirring the fourth solution, the solution is purified so as to obtain a purified colloidal suspension of functionalized nanoparticles.

13. Method of claim 12, wherein the functionalized nanoparticles are isolated from the purified suspension by lyophilization, or the suspension is supplemented with Arabic gum prior to lyophilization.

14. Colloidal suspension comprising functionalized nanoparticles obtainable by a method according to any of claim 1 to 12, for use in a method for treatment of cancer or for use in a method of diagnosis *in vivo* of cancer.

15. Functionalized nanoparticles obtainable by a method according to claim 13, for use in a method for treatment of cancer or for use in a method of diagnosis *in vivo* of cancer.
